# EUROPEAN PATENT APPLICATION

(11) **EP 4 011 332 A1**
(43) Date of publication of application: **15.06.2022**
(21) Application number: 21839309.8
(22) Date of filing: 18.01.2021
(51) Int. Cl.: A61F 2/24

(54) **IMPLANTABLE PROSTHETIC VALVE DEVICE AND IMPLANTATION METHOD THEREFOR**

(30) Priority: 15.10.2020 CN 202011103638
(71) Applicant: Shanghai Trulive Medtech Co., Ltd, Shanghai 201206 (CN)
(72) Inventor: YANG, Wei, Shanghai 201206 (CN); ZHAO, Jing, Shanghai 201206 (CN)
(74) Representative: Argyma
(86) International application number: PCT/CN2021/072484
(87) International publication number: WO 2022/077794

(57) **Abstract**

An implantable prosthesis valve device and a method for implanting the same are provided. The device includes a frame body. The frame body includes a valve leaflet fixing portion for fixing prosthetic valve leaflets, and a first fixing portion located at one end portion of a frame. In an axial direction, the frame body further includes a first buffer portion located between the first fixing portion and the valve leaflet fixing portion, and a second buffer portion located between the other end portion of the frame body and the valve leaflet fixing portion, wherein at least one of the first buffer portion and the second buffer portion has an elasticity in the axial direction.

## Description

### BACKGROUND OF THE INVENTION

### Field of the Invention

The present invention relates to the technical field of medical instruments, in particular, to a prosthesis valve device implanted into a heart.

### Description of the Prior Art

The heart contains four cardiac chambers. The left atrium and the left ventricle are located on the left side of the heart, and the right atrium and the right ventricle are located on the right side of the heart. A ventricle inflow tract is formed between the atrium and the ventricle, the left ventricle and the aorta form a left ventricle outflow tract, and the right ventricle and the pulmonary artery form a right ventricle outflow tract. There is a valve with functions of a "one-way valve" at the left ventricle inflow tract and the right ventricle outflow tract to ensure normal blood flow in the cardiac chambers. When a problem occurs in the valve, the heart hemodynamics changes, and then the heart function is abnormal, resulting in valvular heart disease.

With the development of the social economy and the aging of the population, the incidence of valvular heart disease has increased significantly. Studies have shown that the incidence of valvular heart disease in the elders population over 75 years of age is as high as 13.3%. At present, the traditional surgical treatment is still the preferred treatment for patients with severe valvular lesions. However, but for patients with advanced age, multiple organ diseases, a history of thoracotomy, and/or poor cardiac function, the traditional surgery has disadvantages of high risk and mortality and even no access to surgery for some patients. Since owing advantages such as no need for thoracotomy, small trauma, and quick recovery for patients, the transcatheter mitral valve replacement (TMVR) or the transcatheter mitral valve repair (TMVr) has been widely concerned by experts and scholars.

The valves located at the left ventricle inflow tract and the right ventricle inflow tract are a mitral valve and a tricuspid valve, respectively. These valves are a composite, including valve annulus, valve leaflets, a chordae tendineae, and a papillary muscle; and in some references, also including a ventricle wall. As a support device connecting the mitral valve leaflets and the cardiac muscle, the chordae tendineae is distributed between the valve leaflet and the ventricle wall. The subvalvular structure (chordae tendineae, papillary muscle, ventricle wall, and so forth) of the mitral valve plays an essential role in maintaining the structure and functions of the left ventricle, and the native valve structure is preserved as much as possible during surgery. Therefore, the replacement of the native valve with the prosthetic valve prosthesis implanted by the catheter also requires adaptation to the structure of the native valve, which reduces the damage exerted by the prosthesis valve on the structure of the native valve.

In anatomy, since the size of the valve annulus of the mitral valve is much larger than the size of the valve annulus of the aortic valve, a part of the support body of the prosthesis valve (implanted with the mitral valve) that is used to be matched with the prosthesis valve leaflets requires a larger size for both the circumferential diameter and the axial height. Therefore, the size of the subvalvular prosthesis structure is larger after the prosthesis valve is implanted into the mitral valve, which causes a greater risk of damage on the subvalvular structure of the native valve composite. For a part of patients with mitral regurgitation, there is no calcification part on the valve, and the existing working principle of using the existing radial support force generated between the prosthesis valve and the native valve to prevent the prosthesis valve from displacing may not be applied.

The tricuspid valve, as an atrioventricular valve of the right heart, has a structure similar to the mitral valve. The structure also includes the valve leaflets, the annulus, the chordae tendineae, the papillary muscle, and the cardiac muscle. Therefore, replacing a native mitral valve with a heart valve prosthesis structure can also be applied to replacing a native tricuspid valve, wherein the sizes of the prosthesis valve are different according to different sizes of the native valve.

Although the replacement technique for the mitral valve develops rapidly, there are still some recognized challenges in the design of the valve stent:
1. The mitral valve prosthesis is anchored to the tissue, which may easily cause a larger risk of damage on the native tissues;
2. The anti-fatigue performance of the mitral valve stent is poorer;
3. The anti-fatigue performance of the mitral valve leaflet is poorer.

### SUMMARY OF THE INVENTION

The present invention provides an implantable prosthesis valve device, which may solve the above drawbacks in the prior art.

The technical solution of the present invention is as follows:
An implantable prosthesis valve device includes a hollow, cylindrical frame body, wherein the frame body includes a valve leaflet fixing portion for fixing prosthetic valve leaflets, the prosthetic valve leaflets are fixed to a pre-set position of an inner circumference of the valve leaflet fixing portion, and an end portion of the frame body is a first fixing portion for anchoring; in an axial direction, the frame body further includes a first buffer portion located between the first fixing portion and the valve leaflet fixing portion, and a second buffer portion located between the other end portion of the frame body and the valve leaflet fixing portion, wherein at least one of the first buffer portion and the second buffer portion has an elasticity in the axial direction.

The prosthesis valve device is anchored to a native valve annulus by the first fixing portion, and naturally by the assistance of other anchoring structures playing an anchoring role; the valve leaflet fixing portion is located at an intermediate segment part of the frame body in the axial direction for fixing the prosthetic valve leaflets, and the valve leaflet fixing portion has a certain height in the axial direction. When at least one of the first buffer portion and the second buffer portion has an elasticity in the axial directions, the buffer function in the axial direction may be provided, so that the valve leaflet fixing portion may move axially. The prosthesis valve device, after being implanted into a target position, may reduce an impact force applied on the valve during the blood flushing due to the buffer function of the first buffer portion or the second buffer portion when a heart shrinks and dilates, thereby improving the anti-fatigue performance of the frame body and the endurance property of the valve leaflets.

Since the valve leaflet fixing portion may move axially, a risk of friction between the valve leaflet fixing portion and the native valve annulus is increased, which may cause damages on the native valve annulus of the heart during the long-term use. Therefore, further, an outer circumference of the valve leaflet fixing portion is at least partially configured with a buffer sealing portion to reduce a friction between the frame body and a tissue. The frame body contacts the native valve annulus by the buffer sealing portion, and the friction between the frame body and the native valve annulus is reduced due to the buffering function of the buffer sealing portion when the valve moves up and down, thereby reducing the damages on the tissues, while the buffer sealing portion may further reduce paravalvular leakage.

In some embodiments, the buffer sealing portion is a buffer skirt and distributed uniformly along an outer circumference of the valve leaflet fixing portion; the buffer sealing portion at least includes a first sealing layer and a second layer, wherein the first sealing layer covers an outer surface of the valve leaflet fixing portion, and the second sealing layer covers an outer surface of the first sealing layer. The first sealing layer covering the outer surface of the valve leaflet fixing portion may be used for sealing to prevent paravalvular leakage, and the buffer sealing portion reduces the friction with the tissue by at least double-layered structures.

In some embodiments, the outer surface of the second sealing layer consists of numerous microstructures distributed uniformly, and the microstructures extend outwards and have a compression resilience performance, such as a plush outer surface; when the valve moves up and down, the microstructures of the outer surface may be used for buffering, so as to achieve the effects of reducing the friction between the frame body and the tissue.

In some embodiments, a material of the first sealing layer is selected from materials with good sealing performance, such as at least one of PET and PTFE, and a material of the second sealing layer is selected from materials with a low frictional force, a good sealing performance and better bio-compatibility, such as a plush layer or a velvet ring layer similar to a velour, a velvet, a velveteen and so on.

In some embodiments, the other end portion of the frame body is a second fixing portion, and the first fixing portion and the second fixing portion are used for anchoring respectively, and wherein an included angle between each of the first fixing portion and the second fixing portion and an axis of the frame body is α, α ranging from 45° to 90 ° . The greater the α is, the larger the contact area between the first fixing portion and the second fixing portion and the tissue is; the smaller the α is, the smaller the contact area is; the greater the contact area is, the better the anchoring effect is, and the smaller the obstruction to the blood flow is.

In some embodiments, an included angle β between each of the first buffer portion and the second buffer portion and the axis of the frame body is not greater than the included angle α , so that the resistance of the first buffer portion and the second buffer portion for the blood flow may be reduced, thereby smoothing the blood flow.

In some embodiments, the first buffer portion and the second buffer portion have the elasticity in the axial direction respectively, so as to provide a space for the valve leaflet fixing portion to move up and down in the axial direction. When the first buffer portion and the second buffer portion have the elasticity in the axial direction respectively, the valve leaflet fixing portion may move up and down in the axial direction, thereby providing a buffering function when the heart shrinks or dilates and improving the anti-fatigue performance of the frame body.

In some embodiments, the frame body is made integrally. In some embodiments, the frame body is formed by first manufacturing each parts and then connecting them; the connection is performed by using a connecting media; the connecting media is at least one of a stent structure and a skirt. When the stent structure is used as the connecting media, the connection may be performed by way of welding, glued connection or stitching.

In some embodiments, the first buffer portion or the second buffer portion includes at least one secondary structure, and the secondary structure may be an enclosed ring-shaped structure and disposed co-axially with the valve leaflet fixing portion, wherein one of the secondary structures is formed by connecting two wave-shaped primary structures, and each of the wave-shaped primary structures includes a plurality of crest portions and trough portions; in one of the secondary structures, the crest portion of one wave-shaped primary structure is disposed opposite to the trough portion of the other wave-shaped primary structure, so that the first buffer portion and the second buffer portion acquire the elasticity in the axial direction.

In some embodiments, in one of the wave-shaped primary structures, a pre-set position between one crest portion and the trough portion adjacent to the crest portion is a connecting site, the connection with another wave-shaped primary structure is performed by the connection site, and the connection may be performed by way of weaved connection.

In some embodiments, the first buffer portion or the second buffer portion consists of the plurality of secondary structures, wherein each of the secondary structures extends axially and has the elasticity in the axial direction, and the plurality of secondary structures are distributed uniformly along a circumferential direction of the frame body. The secondary structure may be a wave-shaped structure, or the secondary structure is a spiral structure; when the secondary structure is disposed axially, the first buffer portion and the second buffer portion acquire the elasticity in the axial direction.

In some embodiments, the first buffer portion and the second buffer portion are covered with a skirt respectively, and the skirt is made from a material with the elasticity or the skirt has a pre-set redundancy amount when being covered. The skirt may be used for sealing to some extent, so as to prevent paravalvular leakage.

The present invention further provides a method for implanting a prosthesis valve device including providing an implantable prosthesis valve device, wherein the frame body includes a valve leaflet fixing portion for fixing prosthetic valve leaflets, the prosthetic valve leaflets are fixed to a pre-set position of an inner circumference of the valve leaflet fixing portion, and an end portion of the frame body is a first fixing portion playing an anchoring role; in an axial direction, the frame body further includes a first buffer portion located between the first fixing portion and the valve leaflet fixing portion, and a second buffer portion located between the other end portion of the frame body and the valve leaflet fixing portion, wherein at least one of the first buffer portion and the second buffer portion has an elasticity in the axial direction.

Compared with the conventional art, the present invention has the following beneficial effects:
First, for the implantable prosthesis valve device and the method for implanting the same in the present invention, when at least one of the first buffer portion and the second buffer portion has an elasticity in the axial directions, the valve leaflet fixing portion may move up and down in the axial direction, and a buffering function may be provided when the heart shrinks or dilates, thereby further reducing the force applied on the frame body and improving the anti-fatigue performance of the frame body; meanwhile, a space for buffering the movement exists in the axial direction on the valve, so that the buffer for the movement of the valve leaflets is increased, and the anti-fatigue performance and endurance property of the valve leaflets are improved.
Second, for the implantable prosthesis valve device and the method for implanting the same in the present invention, through the buffering function provided by the buffer sealing portion, the buffer sealing portion may reduce the friction between the valve and the tissue when the valve moves up and down in the axial direction, which reduces the damages on the tissue while functioning as a seal, thereby reducing paravalvular leakage.
Third, for the implantable prosthesis valve device and the method for implanting the same in the present invention, the material with a low frictional force and a good sealing property is used as the material of the buffer sealing portion, so that the damages on the native tissue are minor while reducing paravalvular leakage when the valve moves.
Fourth, for the implantable prosthesis valve device and the method for implanting the same in the present invention, the included angle α between the first fixing portion and the second fixing portion and the axis of the frame body ranges from 45° to 90° , wherein the greater the α is, the larger the contact area between the first fixing portion and the second fixing portion and the tissue is; the smaller the α is, the smaller the contact area is; the greater the contact area is, the better the anchoring effect is, and the smaller the obstruction to the blood flow is; the included angle β between the second buffer portion and the axis of the frame body is not greater than the included angle α , so that the resistance of the first buffer portion and the second buffer portion for the blood flow may be reduced, thereby smoothing the blood flow.

Certainly, any one product for implementing the present invention is unnecessary to achieve all the above advantages at the same time.

### BRIEF DESCRIPTION OF THE DRAWINGS

Fig. 1 is a cross-section diagram of an implantable prosthesis valve device according to Embodiment 1 of the present invention;
Fig. 2 is an integral structural diagram of a buffer sealing portion according to Embodiment 1 of the present invention;
Fig. 3 is a partial structural diagram of the implantable prosthesis valve device according to Embodiment 1 of the present invention;
Fig. 4 is a flatten structural diagram of a first fixing portion or a second fixing portion according to Embodiment 1 of the present invention;
Fig. 5 is a secondary structural diagram of a first buffer portion or a second buffer portion according to Embodiment 1 of the present invention;
Fig. 6 is a flatten structural diagram of the first buffer portion or the second buffer portion according to Embodiment 2 of the present invention;
Fig. 7 is a three-dimensional diagram of a secondary structure according to Embodiment 2 of the present invention.

Reference for numerals: frame body 200; prosthetic valve leaflet 230; valve leaflet fixing portion O; buffer sealing portion 300; first buffer portion B1; second buffer portion B2; first sealing layer 301; second sealing layer 302; first fixing portion A1; second fixing portion A2; secondary structure 400; primary structure 410.

### DESCRIPTION OF THE PREFERRED EMBODIMENTS

In the description of the present invention, it should be noted that "valve" and "prosthesis valve device" have the same meaning. The terms "valve leaflet" and "prosthetic valve leaflet" have the same meaning. The "axial direction" refers to the axial direction of the frame body, and the "outer" refers to a direction facing outward in a radial direction from the center of the frame body. The "height" refers to the size in axial direction, and the "length" refers to the size in radial direction.

In the description of the present invention, it should be noted that the term "velvet" is a generic term for velvet pile fabrics with a plush layer on the surface. The plush layer is mainly composed of the fluff cut by warp. Since the fluffs are in parallel and neat, so the unique luster of velvet is presented. The term "velvet" is named after silk fabrics that form a velvet ring layer or fluff layer on the fabric's surface. The term "flat velvet" is woven with velvet tissue and then cut into velvet, and the surface has a dense, flat, towering, and shiny fluff layer.

In the description of the present invention, it should be noted that orientations or position relationships indicated by terms "center", "upper", "lower", "left", "right", "vertical", "horizontal", "inside", "outside" and the like are orientations or position relationships shown in the drawings, and these terms are merely for facilitating description of the present invention and simplifying the description, but not for indicating or implying that the mentioned device or elements must have a specific orientation and must be established and operated in a specific orientation, and thus, these terms cannot be understood as a limitation to the present invention. Moreover, terms like "first", "second", "third", etc., are only used for description, and should not be considered as a designation or designation of relative importance.

In the description of the present invention, it should be noted that, unless otherwise clearly specified and limited, meanings of terms "install", "connected with", and "connected to" should be understood in a broad sense. For example, the connection may be a fixed connection, a removable connection, an integral connection, a mechanical connection, an electrical connection, a direct connection, an indirect connection by using an intermediate medium, or an intercommunication between two components. For those of ordinary skill in the art, specific meanings of the above terms in the present invention may be understood based on specific situations.

As used in the specification, singular forms "a/an," "one," and "the/that" include plural objects, unless otherwise explicitly stated. As used in the specification, the term "or" is usually used to include the meaning of "and/or", unless otherwise expressly stated.

The following further describes the present invention in combination with specific embodiments.

### Embodiment 1

The present embodiment provides an implantable prosthesis valve device including a hollow, cylindrical frame body 200, wherein the frame body 200 includes a valve leaflet fixing portion O for fixing prosthetic valve leaflets 230. The prosthetic valve leaflets 230 are fixed to a pre-set position of an inner circumference of the valve leaflet fixing portion O. An end portion of the frame body 200 is a first fixing portion A1. In an axial direction, the frame body 200 further includes a first buffer portion B1 located between the first fixing portion A1 and the valve leaflet fixing portion O, and a second buffer portion B2 located between the other end portion of the frame body 200 and the valve leaflet fixing portion O. At least one of the first buffer portion B1 and the second buffer portion B2 has an elasticity in the axial direction. An outer circumference of the valve leaflet fixing portion O is at least partially configured with a buffer sealing portion 300.

The prosthesis valve device is fixed to a native valve annulus by the first fixing portion A1, and naturally, by the assistance of other anchoring structures for anchoring. The valve leaflet fixing portion O is located at an intermediate segment part of the frame body 200 in the axial direction for fixing the prosthetic valve leaflets 230. The valve leaflet fixing portion O has a certain height in the axial direction. The valve leaflet fixing portion O is located between the first buffer portion B1 and the second buffer portion B2. When at least one of the first buffer portion B1 and the second buffer portion B2 has an elasticity in the axial directions, a buffering function in the axial direction may be provided, so that the valve leaflet fixing portion O may move axially. After being implanted into a target position, the prosthesis valve device may reduce an impact force applied by the blood on the frame body 200 when a heart shrinks and dilates due to the buffering function of the first buffer portion B1 or the second buffer portion B2, thereby further improving the anti-fatigue performance of the frame body 200. Meanwhile, a space for buffering the movement exists in the axial direction on the valve, so that the buffer for the movement of the valve leaflets is increased, and the anti-fatigue performance and endurance of the valve leaflets are improved.

The valve leaflet fixing portion O is located at an intermediate segment of the frame body. Since the valve leaflet fixing portion O may move axially, a risk of friction between the frame structure at the valve leaflet fixing portion O and the native valve annulus is increased, which may cause damages on the native valve annulus of the heart during the long-term use. Therefore, further, the valve leaflet fixing portion O of which an outer circumference is at least configured with the buffer sealing portion 300 to reduce a friction between the frame body 200 and a tissue. The frame body 200 contacts the native valve annulus by the buffer sealing portion 300; when the valve moves up and down, the buffer sealing portion 300 may reduce the relative movement between the valve leaflet fixing portion O and the native valve annulus and reduce the friction between the frame structure of the valve and the native annulus, thereby reducing the damages on the tissue. Meanwhile, the valve leaflet buffer portion 300 is distributed at an outer circumference of the valve leaflet fixing portion, which may also function as a seal, thereby reducing paravalvular leakage.

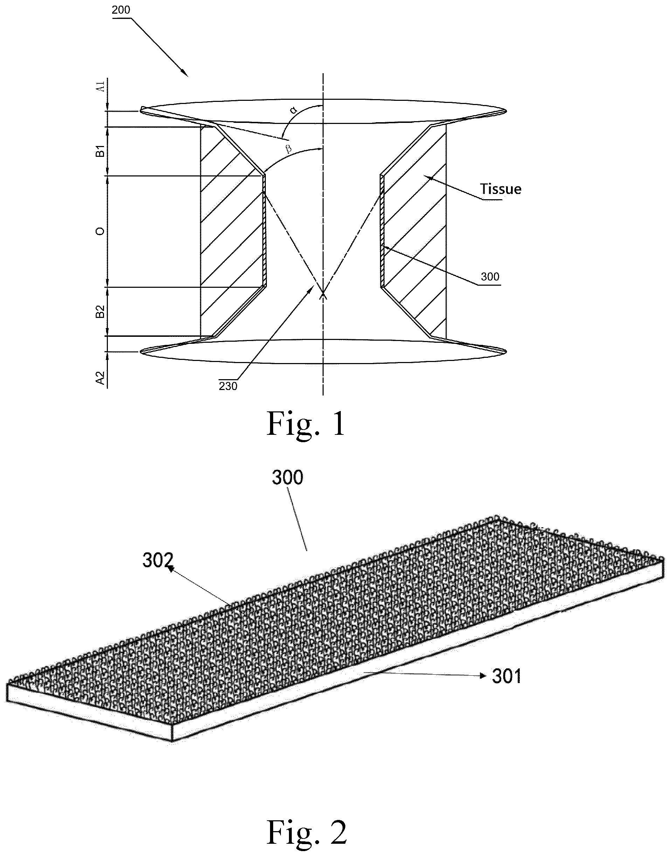

With reference to Figs. 1 to 5, a structural diagram of the implantable prosthesis valve device in the present embodiment is shown.

In the present embodiment, the buffer sealing portion 300 is a buffer skirt, which is distributed uniformly along an outer circumference of the valve leaflet fixing portion O, and may be arranged by way of stitching or covering. The buffer sealing portion 300 at least includes a first sealing layer 301 and a second sealing layer 302, wherein the first sealing layer 301 covers an outer surface of the valve leaflet fixing portion O, and the second sealing layer 302 covers an outer surface of the first sealing layer 301. The first sealing layer 301 covering the outer surface of the valve leaflet fixing portion O may be used for sealing and preventing paravalvular leakage, and the buffer sealing portion 300 reduces the friction with the tissue by double-layered structures. Naturally, in other embodiments, besides the above-mentioned double-layered structures, the buffer sealing portion 300 may also be a structure with three layers, four layers or more layers, which is not repeated here.

In the present embodiment, the second sealing layer 302 is a plush layer with a plush outer surface, and the outer surface of the second sealing layer 302 consists of numerous microstructures distributed uniformly, wherein the microstructures extend outwards and have a compression resilience performance, and specifically, a length of the plush microstructures extending outwards is 0.1 to 2.0mm. The buffer sealing portion 300 provides functions of compression and resilience through the plush outer surface, thereby reaching the effect of reducing the friction between the frame body 200 and the tissue.

Specifically, a material of the first sealing layer 301 is selected from at least one of PET and PTFE, so as to improve the sealing property and reduce paravalvular leakage. A material of the second sealing layer 302 is with a low frictional force and a good sealing performance, so that the second sealing layer 302 has advantages of a low frictional force and a good sealing performance. The second sealing layer 302 specifically may be a velour, a velvet, a velveteen, and so forth. The second sealing layer 302 covers the outermost side of the valve leaflet fixing portion O and contacts the tissue. When the valve moves up and down in the axial direction, the second sealing layer 302 may reduce the friction between the valve and the tissue, while reducing the damages on the tissue.

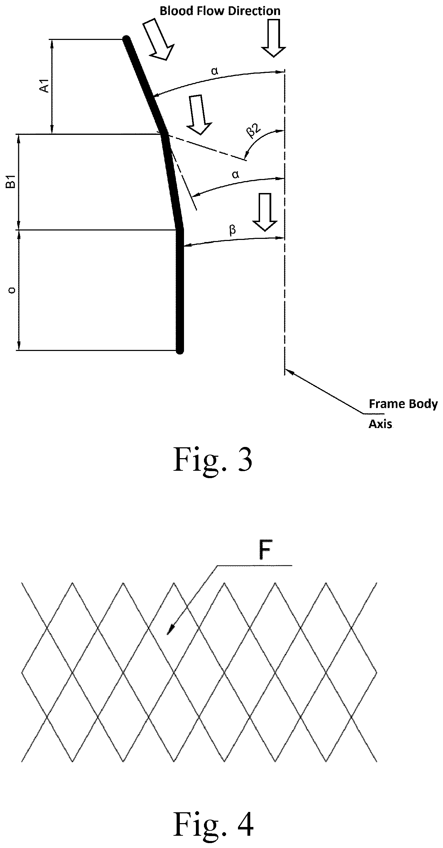

With reference to Fig. 1, in the present embodiment, both two end portions of the frame body 200 are anchoring structures, and the other end portion of the frame body 200 is the second fixing portion A2. After being implanted into the heart, the first fixing portion A1 is located in an atrium as an atrium flange segment, and the second fixing portion A2 is located in the ventricle as a ventricle flange segment, wherein the first fixing portion A1 and the second fixing portion A2 clamp the native tissue (including the native valve leaflets and the native valve annulus) between A1 and A2 to achieve the function of anchoring the valve. The first fixing portion A1 and the second fixing portion A2 are of the same structure, i.e., the flange structure. Specifically, an included angle α between the first fixing portion A1 and the second fixing portion A2 and an axis of the frame body 200 ranges from 45° to 90° . With reference to Fig. 3, a partial structural diagram of the frame body in the present embodiment is shown, wherein the arrow indicates a blood flow direction. The greater the α is, the larger the contact area between the first fixing portion A1 and the second fixing portion A2 and the tissue is; the smaller the α is, the smaller the contact area is; and, the larger the contact area is, the better the anchoring effect is, and the smaller the obstruction to the blood flow is.

Specifically, Fig. 4 is a flatten diagram of an embodiment of a stent structure F of the first fixing portion A1 or a second fixing portion A2, which is formed by staggered uniform arrangement of stent rods, wherein the staggered stent rods form a diamond mesh, and the diamond mesh may change dynamically when the stent rods shrink and expand. Naturally, in other embodiments, the stent structure F may further be a mesh structure formed by other enclosed geometrical shapes (e.g., heart shape, water droplet shape, etc.), which will not be repeated here. The first fixing portion A1 and the second fixing portion A2 are provided with a skirt respectively, wherein the skirt may be distributed on an inner side, an outer side or covered on the inner and outer sides simultaneously of the two fixing portions, and a material of the skirt may be PET or PTFE to reduce paravalvular leakage.

In the present embodiment, there is an included angle β between each of the first buffer portion B1 and the second buffer portion B2 and the axis of the frame body respectively. Preferably, the included angle β is not greater than the included angle α, so that the resistance of the first buffer portion B1 and the second buffer portion B2 for the blood flow may be reduced, thereby smoothing the blood flow. As shown in Fig. 3, when β is smaller than α, the first buffer portion B1 or the second buffer portion B2 has less blockage to the blood flow; when β = β 2 is greater than α, the buffer portions have a significant blockage to the blood flow.

In the present embodiment, the frame body 200 directly contacts the annulus tissue at the valve leaflet fixing portion O, preferably the first buffer portion B1 and the second buffer portion B2 of the frame body have the elasticity in the axial direction respectively to provide a space for the valve leaflet fixing portion O to move up and down in the axial direction, thereby functioning as a buffer.

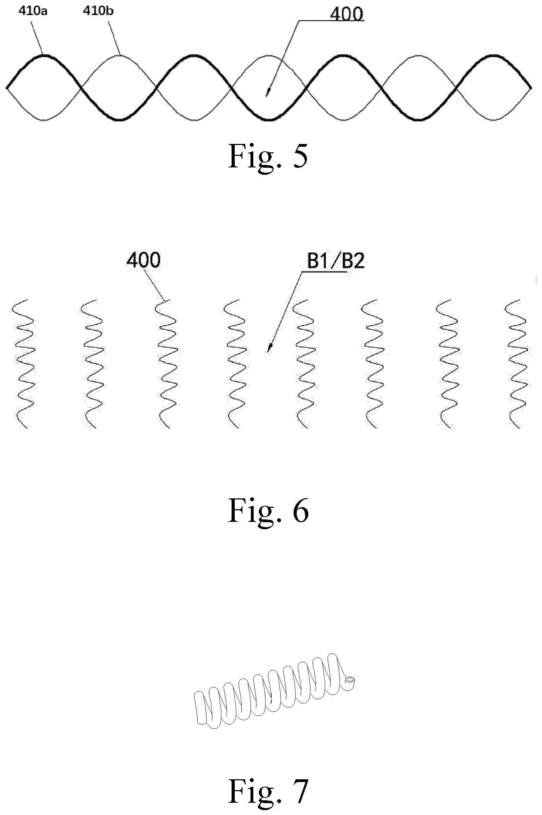

In the present embodiment, the first buffer portion B1 and the second buffer portion B2 are formed by at least one secondary structure 400, e.g., by way of welding, stitching and so on; the secondary structure 400 is an enclosed ring-shaped structure and is disposed co-axially with the valve leaflet fixing portion O. With reference to Fig. 5, a flatten diagram of one secondary structure 400 is shown, wherein one of the secondary structures 400 is formed by connecting two wave-shaped primary structures 410, and each of the wave-shaped primary structures 410 includes a plurality of crest portions and trough portions; in one of the secondary structures 400, the crest portion of one wave-shaped primary structure 410a is disposed opposite to the trough portion of another wave-shaped primary structure 410b, thereby enabling the first buffer portion B1 and the second buffer portion B2 to have the elasticity in the axial direction.

Specifically, in one of the wave-shaped primary structures 410, a pre-set position between the crest portion and the trough portion adjacent to the crest portion is a connection site; in other words, in one of the secondary structures 400, two of the wave-shaped primary structures 410 are connected at respective pre-set connection sites, wherein the connection may be performed by way of weaving, stitching, welding or glued connection.

Further, in the present embodiment, the first buffer portion B1 and the second buffer portion B2 are covered with a skirt, respectively, and the skirt is made from a material with the elasticity or the skirt has a pre-set redundancy when being covered. A region in which the first buffer portion B1 and the second buffer portion B2 located is a buffer region. The skirt may be disposed to cover at least one of an inner side and an outer side of the stent of the buffer region, and the skirt may function as a seal to some extend for preventing paravalvular leakage.

Further, since the first fixing portion A1 and the second fixing portion A2 should function as anchoring, stiffnesses of the first fixing portion A1 and the second fixing portion A2 should be larger than those of other parts of the frame body 200. The first buffer portion B1 and the second buffer portion B2 function as a buffer, with the elasticity in the axial direction, such that stiffnesses of the first buffer portion B1 and the second buffer portion B2 should be smaller than those of other parts of the frame body 200. The valve leaflet fixing portion O is used to support the prosthetic valve leaflets and hence should have a certain stiffness; therefore, the stiffness of the valve leaflet fixing portion O is smaller than the stiffnesses of the first fixing portion A1 and the second fixing portion A2, but is greater than the stiffnesses of the first buffer portion B1 and the second buffer portion B2.

In the present embodiment, the valve leaflet fixing portion O is a mesh structure, such as a diamond mesh structure, and the prosthetic valve leaflets 230 are fixed at the valve leaflet fixing portion O by way of such as stitching, wherein the prosthetic valve leaflets 230 are fixed between two upper and lower ends of the valve leaflet fixing portion O, so that the prosthetic valve leaflets 230 may be prevented from being damaged when the first buffer portion B1 and the second buffer portion B2 generate the compression resilience.

In the present embodiment, the frame body 200 may be made integrally. Naturally, in other embodiments, each part of the frame body 200 (i.e., the first fixing portion A1, the first buffer portion B1, the valve leaflet fixing portion O, the second fixing portion A2 and the second buffer portion B2) may be manufactured respectively and formed by connecting with a connecting media. The connecting media may be the above stent structure F, or the connecting media may be the skirt. When the stent structure is used as the connecting media, the connection may be performed by welding, glued connection, or stitching.

In the present embodiment, each part of the frame body 200 is a frame structure with mesh holes, which is formed by weaving or cutting metal materials, e.g., made from nickel-titanium alloys or other biocompatible materials with shape memory properties, or by an elastic or malleable deformable material such as a balloon-expandable material. The prosthetic valve leaflets 230 dynamically switch between the two states, i.e., an opening state and a closing state. When the prosthetic valve leaflets are in the closing state, the prosthetic valve leaflets 230 are closed or converges by way of sealing and bonding, and the number thereof may be the same as or different from the number of the native valve leaflets. The prosthetic valve leaflets 230 may be: formed by any suitable materials or a combination of materials; prepared by biological tissues such as chemically stable tissues from the heart valves of animals (such as pigs); prepared by pericardial tissues such as cow (bovine pericardium), sheep (ovine pericardium), pig (porcine pericardium), or horses (equine pericardium); or, prepared by small intestinal submucosa (SIS). In addition, synthetic materials may also be selected, such as expanded PTFE or polyester. Optionally, the material further includes thermoplastic polyurethane polyester, polyether urethane, segmented polyether urethane, silicone polyether urethane, silicone-polyurethane polyester, and ultrahigh molecular weight polyethylene. Moreover, the bio-compatible polymer may also be included optionally polyolefin, elastomer, polyethylene glycol, polyethersulfone, polysulfone, polyvinylpyrrolidone, polyvinyl chloride, other fluorine-containing polymers, silicone polyester, siloxane polymers and/or olimers, and/or polycaprolactone, and block copolymers using the same. Optionally, the prosthetic valve leaflets 230 have a surface that is treated by (or reacted with) anticoagulants, and the anticoagulant includes, but is not limited to, heparinized polymers.

In the present embodiment, the frame body 200 is of a cylindrical structure; a cross-section of the frame body 200 may be one of a circle, an oval, a D-shape and a petal shape, or a combination thereof. Two ends of the frame body 200 are flange segments for functioning as an anchor.

In the present embodiment, the first fixing portion A1 and the second fixing portion A2 have the same structure, and the first buffer portion B1 and the second buffer portion B2 have the same structure. The same structure refers to the same size, shape, and tilt angle, i.e., to symmetrical structure. Naturally, in other embodiments, the first fixing portion A1 and the second fixing portion A2 may be of different structures, such as having different sizes or different tilt angles; the first buffer portion B1 and the second buffer portion B2 may also be of different structures, which will not be repeated here.

### Embodiment 2

The present embodiment provides an implantable prosthesis valve device, wherein the implantable prosthesis valve device is similar to that of Embodiment 1 in the structure, but differs from the latter in the structure of the first buffer portion B1 or the second buffer portion B2.

With reference to Fig. 6, a flatten diagram of the first buffer portion B1 or the second buffer portion B2 is shown. In the present embodiment, the first buffer portion B1 or the second buffer portion B2 consists of the plurality of secondary structures 400, wherein each of the secondary structures 400 extends axially and has the elasticity in the axial direction, and the plurality of secondary structures 400 are distributed uniformly along a circumferential direction of the frame body 200.

Specifically, in the present embodiment, the secondary structure 400 is of a spiral structure, as shown in Fig. 7. The plurality of secondary structures 400 are disposed axially, so that the first buffer portion B1 and the second buffer portion B2 acquire the elasticity in the axial direction.

The above disclosure is only the preferred embodiment of the present invention. The preferred embodiments do not describe all the details, and are not intended to limit the invention only to be the specific embodiments. It should be understood that these embodiments are only used for illustrating the present invention, but not for the limitation of the scope of the present invention. In practical application, the improvements and adjustments made by those skilled in the art according to the present invention still fall within the scope of protection of the present invention.

It is evident that various modifications and changes can be made to the content of the specification. The specification selects and specifically describes the embodiments with the purpose of better explain the principle and practical use of the present invention, such that a person ordinarily skilled in the art can well utilize the present invention. The present invention is merely limited by the appended claims and the scope and equivalents thereof.

## Claims

1. An implantable prosthesis valve device, comprising a frame body,
wherein the frame body comprises a valve leaflet fixing portion for fixing prosthetic valve leaflets, the prosthetic valve leaflets are fixed to a pre-set position of an inner circumference of the valve leaflet fixing portion, and one end portion of the frame body is a first fixing portion playing an anchoring role; in an axial direction, the frame body further comprises a first buffer portion located between the first fixing portion and the valve leaflet fixing portion, and a second buffer portion located between the other end portion of the frame body and the valve leaflet fixing portion, wherein at least one of the first buffer portion and the second buffer portion has an elasticity in the axial direction.

2. The implantable prosthesis valve device according to claim 1, wherein an outer circumference of the valve leaflet fixing portion is at least partially configured with a buffer sealing portion to reduce a friction between the frame body and a tissue.

3. The implantable prosthesis valve device according to claim 2, wherein the buffer sealing portion at least comprises a first sealing layer and a second layer, and wherein the first sealing layer covers an outer surface of the valve leaflet fixing portion, and the second sealing layer covers an outer surface of the first sealing layer.

4. The implantable prosthesis valve device according to claim 3, wherein the outer surface of the second sealing layer consists of numerous microstructures distributed uniformly, and the microstructures extend outwards and have a compression resilience performance.

5. The implantable prosthesis valve device according to claim 3, wherein a material of the first sealing layer is selected from at least one of PET and PTFE, and a material of the second sealing layer is a material with a low frictional force and a good sealing property.

6. The implantable prosthesis valve device according to any one of claims 1 to 5, wherein the other end portion of the frame body is a second fixing portion, and the first fixing portion and the second fixing portion are used for anchoring respectively, and wherein an included angle between each of the first fixing portion and the second fixing portion and an axis of the frame body is α , α ranging from 45° to 90° .

7. The implantable prosthesis valve device according to claim 6, wherein an included angle β between each of the first buffer portion and the second buffer portion and the axis of the frame body is not greater than the included angle α .

8. The implantable prosthesis valve device according to any one of claims 1 to 5 or claim 7, wherein the first buffer portion and the second buffer portion have the elasticity in the axial direction respectively, so as to provide a space for the valve leaflet fixing portion to move up and down in the axial direction.

9. The implantable prosthesis valve device according to claim 1, wherein the first buffer portion or the second buffer portion comprises at least one secondary structure, and wherein one of the secondary structures is formed by connecting two wave-shaped primary structures, each of the wave-shaped primary structures comprising a plurality of crest portions and trough portions; in one of the secondary structures, the crest portion of one of the wave-shaped primary structures is disposed opposite to the trough portion of another wave-shaped primary structure.

10. The implantable prosthesis valve device according to claim 9, wherein in one of the wave-shaped primary structures, a pre-set position between one crest portion and the trough portion adjacent to the crest portion is a connecting site.

11. The implantable prosthesis valve device according to claim 1, wherein the first buffer portion or the second buffer portion consists of a plurality of secondary structures, and wherein each of the secondary structures extends axially and has the elasticity in the axial direction, and the plurality of secondary structures are distributed uniformly along a circumferential direction of the frame body.

12. The implantable prosthesis valve device according to claim 1, wherein the first buffer portion and the second buffer portion are covered with a skirt respectively, and the skirt is made from a material with the elasticity or the skirt has a pre-set redundancy amount when being covered.

13. A method for implanting a prosthesis valve device, comprising providing an implantable prosthesis valve device, wherein the frame body comprises a valve leaflet fixing portion for fixing prosthetic valve leaflets, the prosthetic valve leaflets are fixed to a pre-set position of an inner circumference of the valve leaflet fixing portion, and one end of the frame body is a first fixing portion playing an anchoring role; in an axial direction, the frame body further comprises a first buffer portion located between the first fixing portion and the valve leaflet fixing portion, and a second buffer portion located between the other end of the frame body and the valve leaflet fixing portion, wherein at least one of the first buffer portion and the second buffer portion has an elasticity in the axial direction.
